Europäisches Patentamt

European Patent Office

Office européen des brevets

(11) Veröffentlichungsnummer: **0 099 020**
**B1**

(12) **EUROPÄISCHE PATENTSCHRIFT**

(45) Veröffentlichungstag der Patentschrift:
11.05.88

(21) Anmeldenummer: 83106259.1

(22) Anmeldetag: 28.06.83

(51) Int. Cl.⁴: **C 12 N 1/36,** C 12 N 1/26,
C 12 N 1/20, C 02 F 3/34

(54) **Mehrfach substituierte aromatische Kohlenwasserstoffe abbauende Mikroorganismen-Kulturen, Verfahren zu ihrer Herstellung und ihre Verwendung in biologischen Kläranlagen.**

(30) Priorität: 10.07.82 DE 3225885

(43) Veröffentlichungstag der Anmeldung:
25.01.84 Patentblatt 84/4

(45) Bekanntmachung des Hinweises auf die Patenterteilung:
11.05.88 Patentblatt 88/19

(84) Benannte Vertragsstaaten:
BE DE FR GB IT NL SE

(56) Entgegenhaltungen:
DE - A - 2 235 977
FR - A - 1 388 652
FR - A - 2 440 402
GB - A - 2 010 327

TEXTILVEREDLUNG, Band 14, Nr. 11, November 1979,
Seiten 463-466, Basel, CH; H. KULLA et al.: "Aerobe
Abbauversuche mit Azokörpern"
SOVIET INVENTIONS ILLUSTRATED, Woche D51, 3.
Februar 1982, Zusammenfassung Nr. 94390D/51,
London, GB
APPLIED AND ENVIRONMENTAL MICROBIOLOGY,
Band 42, Nr. 1, Juli 1981, Seiten 44-55; C. BRILON et al.:
"Catabolism of naphthalenesulfonic acids by
Pseudomonas sp. A3 and Pseudomonas sp. C22"

(73) Patentinhaber: BAYER AG, Konzernverwaltung RP
Patentabteilung, D-5090 Leverkusen 1 Bayerwerk (DE)

(72) Erfinder: Baumgarten, Jörg, Dr., Henselweg 13,
D-5600 Wuppertal 1 (DE)
Erfinder: Mann, Theo, Dr., Blumenstrasse 18,
D-4018 Langenfeld (DE)
Erfinder: Schmidt, Friedrich, Dr., In den Birken 77,
D-5600 Wuppertal 1 (DE)

## Beschreibung

Bei den bekannten Verfahren der biologischen Abwasserreinigung werden durch den Stoffwechsel von Mikroorganismen die als biologisch leicht abbaubar geltenden Abwasserinhaltstoffe eliminiert. Biologisch als schwer abbaubar geltende, im Abwasser gelöste organische Substanzen werden dabei im allgemeinen, wenn überhaupt, nur in geringem Masse, vor allem durch Fällung und Adsorption, aus dem Abwasser entfernt. Auch durch eine mehrstufige biologische Reinigung wird keine wesentliche Verbesserung für die Elimination biologisch schwer abbaubarer Abwasserinhaltstoffe erreicht.

In Einzelfällen sind spezielle Mikroorganismen bekannt geworden, die schwer abbaubare Stoffe in ihrem Stoffwechselprozess abbauen können. So sind z.B. Organismen bekannt, die einfache Naphthalinsulfonsäuren oder Chloraromaten abzubauen vermögen. Jedoch kommen solche speziellen Mikroorganismen in den üblichen Kläranlagen nicht vor. Dies ist einerseits bedingt durch die geringe Vermehrungsrate dieser Organismen, die bei Anwesenheit von biologisch gut abbaubaren Substanzen von den anderen anwesenden Mikroorganismen überwachsen werden und darüber hinaus eine Abbaurepression bei den speziellen Organismen für die Elimination der schwer abbaubaren Verbindungen durch leicht abbaubare erfolgt.

Andererseits können diese speziellen Organismen wegen ihrer schlechten Flockbarkeit bzw. ihrer schlechten Aufwachseigenschaft kaum im System zurückgehalten werden. Sie schwemmen aus. Es wurde daher bereits vorgeschlagen, solche Mikroorganismen auf geeigneten Substraten zu fixieren, so dass sie im System erhalten bleiben.

Wegen der hohen Eigentoxizität, der geringen Abbauleistung der Mikroorganismen und der Entstehung toxischer Abbauprodukte ist der Abbau solcher schwer abbaubaren Stoffe im allgemeinen nicht über das Laborstadium hinausgekommen. Eine Ausnahme bilden hier Chloraromaten, für die leicht adaptierbare Mikroorganismen in der Natur vorhanden sind.

Als besonders problematisch im Hinblick auf den mikrobiologischen Abbau gelten mehrfach substituierte Naphthaline, insbesondere Naphthalinsulfonsäuren, die sogenannten Buchstabensäuren. Wenn in Einzelfällen ihre Entfernung aus Kulturflüssigkeiten mit Mischpopulation gelang, wird dies auf intracelluläre Akkumulation zurückgeführt.

Aufgabe der vorliegenden Erfindung ist es zunächst, Mikroorganismen aufzufinden, die mit Buchstabensäuren als einziger Kohlenwasserstoff-Quelle vermehrt werden können. Dabei wurde ein neuer Konstruktionsweg für Mikroorganismen aufgefunden, mit dem solche Mikroorganismen hergestellt werden können. Jedoch waren die so hergestellten Mikroorganismen noch nicht für den Einsatz in biologischen Kläranlagen geeignet, da der Abbau unter gleichzeitiger Ausscheidung von z.T. toxischen Zwischenprodukten erfolgte.

Aufgabe der vorliegenden Erfindung ist daher weiterhin, die Mikroorganismen so weiterzuentwickeln, dass sie einen Totalabbau der Buchstabensäuren bewirken können. Es wurde gefunden, dass dies durch einen weiteren Adaptionsschritt gelingt, bei dem die Mikroorganismen auf speziell präparierten Festkörperoberflächen fixiert sind.

Schliesslich wurde gefunden, dass sich das erfindungsgemässe Konstruktionsprinzip so verallgemeinern lässt, dass für eine Vielzahl schwer abbaubarer, mehrfach verschieden substituierter Kohlenwasserstoffe Mikroorganismen-Kulturen hergestellt werden können, die mit diesen mehrfach verschieden substituierten Kohlenwasserstoffen als einziger Kohlenwasserstoff-Quelle unter Totalabbau vermehrt werden können.

Es wurde ferner gefunden, dass die erhaltenen Mikroorganismen-Kulturen auch in Mischkulturflüssigkeiten, wie sie z.B. in biologischen Kläranlagen vorliegen, stabil bleiben und ihre Fähigkeit zum Totalabbau behalten.

Gegenstand der vorliegenden Erfindung sind daher mehrfach substituierte Kohlenwasserstoffe mit nicht identischen Substituenten total abbauende Mikroorganismen-Kulturen, bestehend aus an positive Ladungen tragenden Festkörperoberflächen anhaftenden Mikroorganismen, wobei die Mikroorganismen mit diesen mehrfach substituierten aromatischen Kohlenwasserstoffen als einziger Kohlenwasserstoffquelle vermehrt werden können.

Als Substituenten im Sinne der vorliegenden Erfindung zählen anorganische Säurereste wie $-CN-NO_2$, $-Cl$, $-SO_3^-$, Aminreste, Aliphaten, wie z.B. Methyl, Ethyl oder substituierte Aliphaten. Ferner können OH-Gruppen oder COOH-Gruppen vorhanden sein. Bei aliphatischen Kohlenwasserstoffen und einfachen Ringsystemen sollen unter «mehrfach substituiert» OH-, COOH- und Alkyl-O-Gruppen nicht mitgezählt werden. In diesem Sinne soll Nitrophenol als einfach substituiert gelten, obwohl eine OH-Gruppe als zweiter Substituent vorhanden ist. Entsprechend gilt Nitrokresol als zweifach substituiert und Chlornitrokresol als dreifach substituiert. Dagegen sollen bei mehrfach cyclischen Systemen wie substituierten Naphthalinen unter «mehrfach substituiert» OH- und COOH-Gruppen mitgezählt werden. Zum Beispiel soll Hydroxynaphthalinsulfonsäure als zweifach substituiert gelten.

Diese Zählweise der Substituenten hat ihren Grund darin, dass bei den einfacher aufgebauten Verbindungen der mikrobiologische Abbau durch OH- und COOH-Gruppen im allgemeinen nicht erschwert wird. Diese Substituenten können daher bei der Auswahl spezieller Mikroorganismen ausser Betracht bleiben.

Nicht identische Substituenten im Sinne der vorliegenden Erfindung liegen dann vor, wenn unter den «zählenden» Substituenten mindestens zwei verschiedene Substituenten vorhanden sind. Zum Beispiel gilt ein N-fach substituierter Kohlenwasserstoff dann als nicht identische Substituenten aufweisend, wenn höchstens N – 1-Substituenten identisch sind.

Mehrfach substituierte Kohlenwasserstoffe im Sinne der vorliegenden Erfindung sind mindestens zweifach, vorzugsweise zwei- bis zehnfach und insbesondere dreifach substituierte Kohlenwasserstoffe im Sinne der o.g. Zählung.

Als Kohlenwasserstoffe kommen aliphatische und aromatische Kohlenwasserstoffe in Frage, bis hin zu höhermolekularen wasserlöslichen Verbindungen, wie sie bei der Herstellung von Pflanzenschutzmitteln und Farbstoffen vorkommen.

Als zweifach substituierte Benzole bzw. Phenole sind Nitrochlorbenzol, Nitrochlorphenol, Nitrokresol, Nitrotoluol, Nitroanilin, und Aminonitrophenol zu nennen. Als dreifach substituierte Benzole bzw. Phenole sind Chlornitrokresol, Chlornitrotoluol und Chlornitroanilin zu nennen.

Als substituierte Mehrfachringsysteme sind vor allem Naphthaline zu nennen, insbesondere Naphthalinsulfonsäure, z.B. Hydroxynaphthalinsulfonsäure, Amino-naphthalin-sulfonsäuren, Amino-hydroxy-naphthalin-sulfonsäuren und Amino-naphthalin-disulfonsäuren.

Als Trägermaterialien, die Festkörperoberflächen mit positiven Ladungen aufweisen, können Ionenaustauscher, geladene Polyurethane, Polystyrol oder Trägermaterialien mit speziell präparierten Oberflächen wie Polyethylenkugeln, die mit einem positiv geladenen Polyelektrolyten (z.B. Prestaminol) behandelt wurden, eingesetzt werden. Es können aber auch gelagerte Trägermaterialien wie z.B. mit Aluminiumverbindungen behandelte $SiO_2$-Gele oder -Sole oder gelartige Polyisocyanate eingesetzt werden. Wesentlich ist, dass oberflächlich positive Ladungen vorhanden sind.

Die Auswahl der Trägermaterialien erfolgt in Abhängigkeit von dem beabsichtigten Einsatz der erfindungsgemässen Mikroorganismen-Kulturen. Zum Beispiel ist es für ein Tropfkörperverfahren zweckmässig, die Mikroorganismen-Kulturen in Form von auf behandelte Polyethylenkugeln aufgebrachte Mikroorganismen einzusetzen.

Gegenstand der vorliegenden Erfindung ist auch ein Verfahren zur Herstellung von mindestens N-fach substituierte Kohlenwasserstoffe mit nicht identischen Substituenten abbauenden Mikroorganismen-Kulturen, das dadurch gekennzeichnet ist, dass

a) zunächst verschiedene, höchstens (N – 1)-fach substituierte Kohlenwasserstoffe abbauende Mikroorganismen mit diesen höchstens (N – 1)-fach substituierten Kohlenwasserstoffen als Kohlenwasserstoff-Quelle getrennt vermehrt werden,

b) wobei die Substituenten der höchstens (N – 1)-fach substituierten Kohlenwasserstoffe gemeinsam mindestens alle Substituenten der N-fach substituierten Kohlenwasserstoffe mindestens einmal aufweisen,

c) die verschiedenen, an höchstens (N – 1)-fach substituierte Kohlenwasserstoffe adaptierten Kulturen vereinigt werden und durch allmählichen Austausch des Substrates durch den N-fach substituierten Kohlenwasserstoff an diesen adaptiert werden,

d) die N-fach substituierte Kohlenwasserstoffe abbauenden Mikroorganismen auf positive Ladungen tragende Trägermaterialien aufgebracht werden und

e) unter Zugabe des N-fach substituierten Kohlenwasserstoffs weiter vermehrt werden.

Bei dem erfindungsgemässen Verfahren handelt es sich demnach um ein Verfahren, das aus drei Adaptionsschritten besteht.

Das Verfahren geht aus von Mikroorganismen, die geringer substituierte Kohlenwasserstoffe abzubauen vermögen. In einem ersten Adaptionsschritt (oben genanntes Merkmal a) werden diese Mikroorganismen einem erhöhten Selektionsdruck dadurch ausgesetzt, dass sie mit den geringer substituierten Kohlenwasserstoffen als Kohlenstoff-Quelle vermehrt werden. Dabei werden mehrere, vorzugsweise mindestens zwei verschiedene Populationen mit verschieden substituierten Kohlenwasserstoffen getrennt vermehrt.

Vorzugsweise soll der Kohlenwasserstoff, an dessen Abbau adaptiert wird, einen merklich, besonders bevorzugt den überwiegenden Teil des Substrates bilden, sodass ein ausreichender Selektionsdruck ausgeübt wird.

In einem zweiten Adaptionsschritt (oben genanntes Merkmal c) werden diese verschiedenen Populationen vereinigt und an den höher substituierten Kohlenwasserstoff adaptiert und vermehrt. Es wird angenommen, dass in diesem Adaptionsschritt zwischen den vorher getrennt vermehrten Mikroorganismen durch Plasmidübertragung Abbaueigenschaften übertragen werden. Selektive Adaption dürfte in diesem Adaptionsschritt höchstens von untergeordneter Bedeutung sein. Es wird eine Mikroorganismen-Population erhalten, die die höher substituierten Kohlenwasserstoffe zwar abbauen kann, jedoch Zwischenprodukte des Abbaus ausscheidet.

Im dritten Adaptionsschritt (oben genanntes Merkmal e) werden schliesslich Mikroorganismen-Kulturen erhalten, die den Totalabbau der höher substituierten Kohlenwasserstoffe bewirken. Es ist nicht bekannt, welche Veränderungen die Mikroorganismen-Kultur in diesem dritten Adaptionsschritt erfährt. Jedoch wird vermutet, dass eine Art katalytische Aktivierung der Mikroorganismen erfolgt. Diese katalytische Aktivierung erfolgt offenbar nicht spontan, sondern benötigt eine gewisse Gewöhnungszeit, die zwischen wenigen Tagen und einem Monat betragen kann.

Bei dem erfindungsgemässen Verfahren zur Herstellung der höher substituierte Kohlenwasserstoffe abbauenden Mikroorganismen kann von bekannten, die einzusetzenden niedriger substituierte Kohlenwasserstoffe abbauenden Mikroorganismen ausgegangen werden, soweit diese zur Verfügung stehen.

Stehen die niedriger substituierte Kohlenwasserstoffe abbauenden Mikroorganismen nicht zur Verfügung, kann a priori begonnen werden. Hierzu werden in bekannter Weise Proben von Oberflächengewässern, aus Kläranlagen oder Waldböden eingesetzt, aus denen durch Vermehrung unter Zugabe von Nährlösungen und Separation Mischpopulationen gezüchtet werden. Aus diesen Mischpopulationen werden in bekannter Weise durch Selektionsverfahren Mikroorganismen gewonnen, die die entsprechenden nicht oder nur einfach substituierten Kohlenwasserstoffe abbauen.

Aus den entsprechenden nicht substituierte Kohlenwasserstoffe abbauenden Mikroorganismen gelingt es im allgemeinen in aufeinanderfolgenden Adaptionsschritten Mikroorganismen zu gewinnen, die zunächst einfach substituierte Kohlenwasserstoffe und danach zweifach substituierte Kohlenwasserstoffe abzubauen vermögen.

Liegen bereits verschieden einfach substituierte Kohlenwasserstoffe abbauende Mikroorganismen vor, können die zweifach substituierte Kohlenwasserstoffe abbauenden Mikroorganismen vorteilhaft nach dem erfindungsgemässen Verfahren gewonnen werden.

Das erfindungsgemässe Verfahren kann demnach mehrfach wiederholt werden, indem zunächst aus einfach substituierte Kohlenwasserstoffe abbauenden Mikroorganismen zweifach substituierte Kohlenwasserstoffe abbauende Mikroorganismen gewonnen werden, aus diesen zweifach substituierte Kohlenwasserstoffe abbauenden Mikroorganismen durch Wiederholung des erfindungsgemässen Verfahrens dreifach substituierte Kohlenwasserstoffe abbauende Mikroorganismen gewonnen werden. Allgemein gelingt es, aus (N – 1)-fach substituierte Kohlenwasserstoffe abbauenden Mikroorganismen N-fach substituierte Kohlenwasserstoffe abbauende Mikroorganismen zu gewinnen.

Obwohl es erfindungsgemäss bevorzugt ist, zur Gewinnung von N-fach substituierte Kohlenwasserstoffe abbauenden Mikroorganismen von (N – 1)-fach substituierte Kohlenwasserstoffe abbauenden Mikroorganismen auszugehen, ist dies jedoch nicht in jedem Fall notwendig. Zum Beispiel gelingt die Herstellung von 3-fach substituierte Kohlenwasserstoffe abbauenden Mikroorganismen in bestimmten Fällen auch dann, wenn von einem zweifach substituierte Kohlenwasserstoffe abbauenden und einem einfach substituierte Kohlenwasserstoffe abbauenden Mikroorganismus ausgegangen wird. Wesentlich ist lediglich, dass alle Substituenten im ersten getrennten Adaptionsschritt vorhanden sind.

Obwohl es ferner erfindungsgemäss bevorzugt ist, zur Herstellung mehrfach substituierte Kohlenwasserstoffe abbauender Mikroorganismen von jeweils geringer substituierte Kohlenwasserstoffe abbauenden Mikroorganismen auszugehen, wobei die Kohlenwasserstoffe mit Ausnahme der Substituenten identisch sind, ist dies nicht in jedem Fall notwendig und möglich. Wesentlich ist, dass mit dem geringer substituierten Kohlenwasserstoff die Mikroorganismen an einen identischen ersten Abbauschritt adaptiert werden. So kann z.B. zur Herstellung von mehrfach substituierten komplizierten Ringsystemen abbauenden Mikroorganismen für den ersten Adaptionsschritt auch ein niedriger substituiertes weniger kompliziertes Ringsystem als Substrat vorgesehen werden. Zum Beispiel gelingt es aus an Naphthalinsulfonsäure und an Anilin im ersten Adaptionsschritt getrennt adaptierte Mikroorganismen im zweiten Adaptionsschritt Aminonaphthalinsulfonsäure abbauende Mikroorganismen zu erhalten, die dann im dritten Adaptionsschritt weiter für den Totalabbau adaptiert werden können.

Erfindungsgemäss wird im zweiten Adaptionsschritt bevorzugt von zwei getrennt an den Abbau von niedriger substituierten Kohlenwasserstoffen adaptierten Mikroorganismen ausgegangen, wobei die zwei verschieden substituierten Kohlenwasserstoffe bereits alle Substituenten des höher substituierten Kohlenwasserstoffs enthalten. Jedoch ist nicht ausgeschlossen, dass der zweite Adaptionsschritt an z.B. dreifach substituierte Kohlenwasserstoffe auch dann gelingt, wenn von drei nur einfach unterschiedlich substituierte Kohlenwasserstoffe abbauenden Mikroorganismen ausgegangen wird.

Am einfachsten gelingt der zweite Adaptionsschritt dann, wenn der den Abbau am meisten behindernde Substituent bereits in jedem der Substrate des ersten Adaptionsschrittes vorhanden war. So geht man z.B. vorteilhaft von substituierte Naphthalinsulfonsäuren abbauenden Mikroorganismen aus, wenn man höher substituierte Naphthalinsulfonsäure abbauende Mikroorganismen gewinnen will, da die Sulfonsäuregruppe in dieser Substanzklasse den Abbau am stärksten behindert.

Auf die erfindungsgemäss wesentliche Bedeutung des dritten Adaptionsschritts wurde bereits hingewiesen. Wesentlich für den dritten Adaptionsschritt ist, dass die auf das Trägermaterial aufgebrachten Mikroorganismen auf spezifischem Substrat adaptiert werden. Dieser dritte Adaptionsschritt geling z.B. nicht, wenn die Mikroorganismen-Kultur, d.h. Mikroorganismen mit Trägersubstanz, in eine biologische Kläranlage eingebracht wird, auch wenn diese die speziellen mehrfach substituierten Kohlenwasserstoffe enthält.

Es wurde jedoch gefunden, dass verschiedene, erfindungsgemäss erhaltene, verschiedene mehrfach substituierte Kohlenwasserstoffe abbauende Mikroorganismen gleichzeitig auf Trägerkörper aufgebracht werden können und gemeinsam dem dritten Adaptionsschritt unterzogen werden können, wenn ein entsprechend abgestimmtes Mischsubstrat als gemeinsame Kohlenwasserstoffquelle für die verschiedenen Mikroorganismen eingesetzt wird. Das Mischsubstrat soll die substituierten Kohlenwasserstoffe enthalten, auf denen die Mirkoorganismen im zweiten Adaptionsschritt getrennt gezüchtet werden. Darüber hinaus ist die Anwesenheit einer gewissen Menge anorganischer Salze vorteilhaft. Ferner können noch substituierte Kohlenwasserstoffe, die in verschiedenen ersten Adaptionsschritten als Substrat eingesetzt waren, in dem Mischsubstrat enthalten sein. Das Mischsubstrat soll jedoch nicht solche Substanzen in merklicher Konzentration, z.B. mehr als 20%, enthalten, die in üblichen Abwässern zum BOD beitragen. Vorzugsweise hat das Mischsubstrat die Zusammensetzung der biologisch abzubauenden, schwer abbaubaren Substanzen im Abwasser ohne Berücksichtigung der BOD-Substanzen.

Die erhaltenen Mikroorganismen-Kulturen, bestehend aus Mikroorganismen und Trägermaterial, sind als solche transport- und lagerfähig. Nach längerer Lagerzeit ist lediglich eine Reaktivierung auf dem entsprechenden Substrat als Nährlösung zweckmässig.

Sind die Mikroorganismen-Kulturen einmal vorhanden, so reicht es für ihre Vermehrung, in eine entsprechende Nährlösung als Substrat, in der sich die entsprechenden Mikroorganismen-Kulturen (Trägermaterial plus Mikroorganismen) befinden, zusätzlich Trägermaterialien einzuführen, auf die dann die entsprechenden Mikroorganismen aufwachsen. Die Vermehrung kann demnach in einem kontinuierlichen Reaktor erfolgen, in dem Trägermaterialien und Nährlösung unter Belüftung eingeführt werden und kontinuierlich Mikroorganismen-Kulturen zum Einsatz in biologischen Kläranlagen entnommen werden.

Die Erfindung wird anhand der nachfolgenden Beispiele weiter erläutert:

Beispiel 1:
Gewinnung von Mikroorganismenkulturen zum Abbau von mehrfach substituierten Naphthalinsulfonsäuren

Als Ausgangskultur zur Herstellung einer Mikroorganismenkultur, die mehrfach substituierte Naphthalinsulfonsäuren abbauen kann, dienten zwei Pseudomonas-Stämme A 3 und C 22. (H.J. Knackmuss, App. Environ Microbiol., Vol. 42, p 44–55, (1981). Diese zum Abbau von einfach substituierten Naphthalinsulfonsäuren befähigten Stämme (Abbau von Naphthalin-1-Sulfonsäure (NS-1) und Naphthalin-2-Sulfonsäure (NS-2) wurden mit Belebtschlamm einer Kläranlage der chemischen Industrie (Bayer AG, Dormagen/Bayer AG, Leverkusen) kombiniert und in Erlenmeyerkolben mit je 0,1% NS-1 und NS-2 angezogen. Der Belebtschlamm war in der Lage, einfach phenolische Aromaten abzubauen.

Die Adaption dieses Kulturgemisches an zweifach substituierte Naphthalinsulfonsäuren erfolgte im 10 1-Massstab (Fermenter Fa. Braun-Melsungen). Die Nährlösung enthielt neben je 0,1% NS-1. NS-2, Naphthalin-1,6-disulfonsäure (NS-1,6), Naphthalin-2,6-disulfonsäure (NS-2,6) und 6-Hydroxy-2-naphthalinsulfonsäure (Schäffersäure). Als zusätzliche Wachstumssubstrate dienten je 0,05% Ethanol und Acetat.

Nach dem Anwachsen des Kulturgemisches wurde in kontinuierlicher Kultur die Züchtung für den Abbau von zweifach substituierten Naphthalinsulfonsäuren begonnen. Die Verweilzeit der Nährlösung betrug am Anfang 50 Stunden. Sie wurde über etwa 3 Monate beibehalten und dann innerhalb eines Monats schrittweise auf 20 h verringert. Der Abbau der zweifach substituierten Naphthalinsulfonsäuren betrug dann mehr als 80%.

Die kontinuierliche Fermentation dieser Kulturen mit einer der Nährlösung zugesetzten Amino-Naphthalinsulfonsäure, 7-Amino-4-hydroxy-2-naphthalinsulfonsäure (I-Säure), brachte lediglich Abbauwerte unter 50%.

Eine Kultur, die Naphthalinsulfonsäure mit zusätzlichem Aminorest abbaut, wurde durch gemeinsame Züchtung mit einer Anilin-abbauenden Kultur gewonnen, wobei die Anilinkultur aus Klärschlamm von Kläranlagen der chemischen Industrie durch Adaption an 100 ppm Anilin stammte. Die Naphthalinsulfonsäure-Kultur wurde mit der Anilin-abbauenden Kultur gemeinsam in einem 10 1-Fermenter gezogen.

Die Nährlösung enthielt

| | |
|---|---|
| NS-2 | 200 ppm |
| NS-1 | 200 ppm |
| Schäffersäure | 200 ppm |
| Anilin | 100 ppm |
| Gamma-Säure (6-Amino-4-hydroxy-2-naphthalinsulfonsäure) | 50 ppm |

Nach dem Anwachsen der kombinierten Kultur wurde mit einer Verweilzeit von 20 h die kontinuierliche Züchtung begonnen. Nach 10 Tagen wurden etwa 85–90% der Naphthalinsulfonsäuren abgebaut. Dieser Abbauwert blieb über mehrere Monate konstant. Danach wurde 50 ppm I-Säure und nach 7 Tagen 100 ppm I-Säure der Nährlösung zugesetzt. Der Abbau ging auf 70–80% zurück, blieb aber über ein Jahr bei kontinuierlicher Züchtung konstant. Die Verschlechterung des Abbaues um 10–20% war auf die Bildung und Anhäufung von Zwischenprodukten des Abbaues von Aminonaphthalinsulfonsäuren zurückzuführen. Diese Substanzen führen zu einer intensiven Braunfärbung des Kulturmediums.

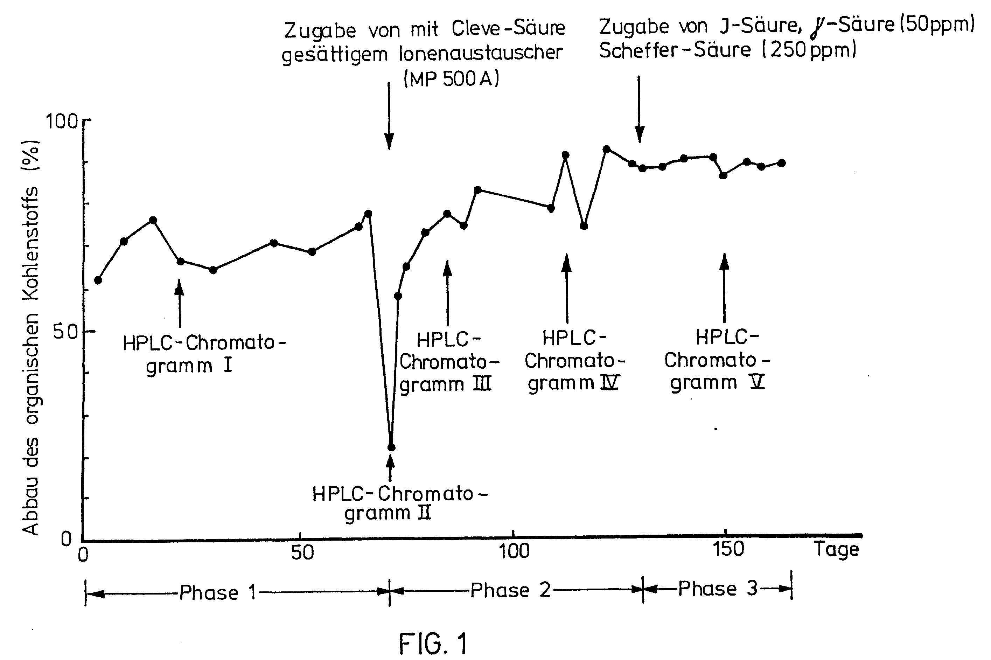

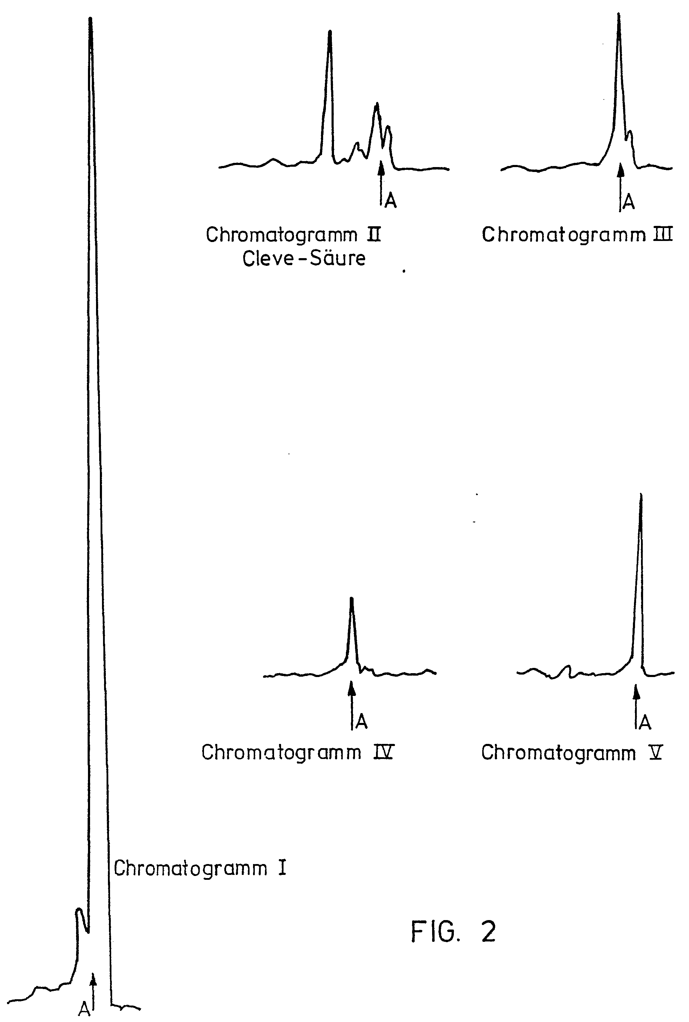

Die weitere Entwicklung der Kultur erfolgte in 1 1-Erlenmeyerkolben. Diese weitere Entwicklung wird anhand der Figuren 1 und 2 gleichzeitig näher erläutert:

Fig. 1 zeigt den Verlauf des Abbaues des organischen Kohlenstoffs (TOC) während der weiteren Entwicklung.

Fig. 2 zeigt Chromatogramme eines Abbauproduktes von Clevesäure von bei in Fig. 1 angegebenen Zeitpunkten der Entwicklung entnommenen Proben. Die Nährlösung enthielt:

| | |
|---|---|
| NS-1 | 250 ppm |
| NS-2 | 250 ppm |
| Anilin | 250 ppm |
| Clevesäure-1,7 (8-Amino-2-naphthalinsulfonsäure) | 250 ppm |

Die Kultur wurde zunächst batchweise unter Schütteln angezogen, wobei alle 2–5 Tage die Nährlösung erneuert wurde.

Unter diesen Bedingungen wurde 75 Tage kultiviert. Der Abbau der organischen Komponenten lag bei etwa 70% (Fig. 1, Phase 1). Der Rest war ein Abbauprodukt der Clevesäure-1,7, das mittels HPLC-Analytik als neue Substanz erfasst werden konnte. (Fig. 2, Chromatogramm I, Peak A). Nach 75 Tagen Züchtung der kombinierten Anilin-Aminonaphthalinsulfonsäure-Kultur wurden zu 100 ml Nährlösung 10 ml mit Clevesäure gesättigter kationischer Ionenaustauscher (Levatit MP 500 A) zugesetzt. Der Abbau steigerte sich nach 40tägiger Inkubation mit 15maligem Überimpfen in frische Nährlösung auf etwa 90% (Fig. 1, Phase 2). Das Zwischenprodukt des Abbaues trat nur noch in geringen Konzentrationen auf (Fig. 2, Chromatogramm III).

Die Kultur war nach dieser Züchtungszeit auch in der Lage, I-Säure und Gamma-Säure vollständig abzubauen (Fig. 1, Phase 3). Eine Zugabe von 50 ppm der beiden Säuren veränderte die Abbauwerte nur wenig (Fig. 2, Chromatogramm V). In dieser Phase der Züchtung konnte auf Anilinzusatz zur Nährlösung verzichtet werden.

Die verschiedenen Stufen dieses Beispiels sind in der nachfolgenden Tabelle 1 noch einmal dargestellt.

Tabelle 1

Züchtungsphasen bei der Herstellung von mikrobiellen Kulturen zum Abbau von Naphthalinsufonsäure mit 3 Resten

| Züchtungsphasen (Nr. d. Kultur) | Ausgangs-kultur | Adaptierte Kultur zum Abbau von | Adaptions-zeit | Abbauleistung | Nährlösungs-Komponenten z. Anzucht | Kultivierungs-art |
|---|---|---|---|---|---|---|
| 1 | A3, C22 | NS-1, NS-2 | 0 | total | NS-1, NS-2 | batch |
| 2 | Klärschlamm | Phenole | 0 | total | p-Nitrophenol Phenol | batch |
| 3 | Kultur 1 + Kultur 2 | NS-1, NS-2,6 NS-2, NS-1,6 Schäffersäure | 4 Monate | 80% | NS-1, NS-2, NS-2,6 NS-1,6, Schäffersäure | kontinuierlich |
| 4 | Klärschlamm | Anilin | 1 Monat | total | Anilin | batch |
| 5 | Kultur 3 + Kultur 4 | I-Säure Gamma-Säure Clevesäure | 1 Monat | 75% Ausscheidung v. Produkten des Abbaues | I-Säure, Anilin Gammasäure | kontinuierlich |
| 6 | Kultur 5 | wie 5 | 1,5 Mon. | mehr als 90% o. Ausscheidg. v. Produkten des Abbaues | Ionenaustauscher MP500A NS-1, NS-2, Anilin Clevesäure I-Säure Gamma-Säure | batch mit zweitägiger Überimpfung |
| 7 | Kultur 5 | wie 5 | 0 | 90% | wie 6, aber ohne Anilin | wie 6 |

**Beispiel 2**
Gewinnung von Mikroorganismenkulturen für den Abbau mehrfach substituierter Aromaten

Es wurde von Kulturen ausgegangen, die einfach substituierte, aromatische Verbindungen abbauen können.

Die Kulturen wurden aus Klärschlamm von Kläranlagen der chemischen Industrie und Kulturböden mit üblichen Anreicherungstechniken gewonnen. Ausserdem wurden Bakterienstämme aus Kulturensammlungen (DSM), wie Pseudomonas putida. Ps. spec B 13, Acinetobacter, Arthrobacter verwendet. Diese Kulturen bauen einfache, aromatische Verbindungen, wie 4-Nitrophenol, 2-Chlorbenzoat, Anilin und Aminobenzoat ab. Zur Gewinnung von Kulturen, die mehrfach substituierte aromatische Verbindungen abbauen können, werden diese Kulturen vereinigt und in zwei verschiedenen Nährlösungen weitergezüchtet. Nährlösung 1 enthielt neben den einfach substituierten Verbindungen noch 1-Chlor-4-nitrobenzol, 2-Nitrom-xylol und 1,3-Dinitrobenzol. Nährlösung 2 enthält zusätzlich 4-Nitrohydroxitoluol und 2,4-Dinitrophenol und Nitroanilin.

Die Anzucht erfolgte in 2 l-Fermentern.
Nach 8-tägiger batch-Kultur wurde die Züchtung in kontinuierlicher Kultur weitergeführt. Die Konzentrationen der mehrfach substituierten Verbindungen in der Nährlösung wurde von anfangs 20 ppm in Intervallen auf 100 ppm erhöht. Nach etwa viermonatiger kontinuierlicher Kultur wurden die mehrfach substituierten Verbindungen in Konzentrationen über 100 ppm vollständig abgebaut.

Die auf diese Weise gewonnenen Kulturen wurden vereinigt und zur biologischen Reinigung von Produktionsabwässern der chemischen Industrie eingesetzt.

Folgende Abwässer wurden mit den angegebenen Abbauwerten überprüft.

| Abwasser 1 | Nitrobenzol | 25% |
|---|---|---|
| Abwasser 2 | Nitrotoluol | 35% |
| Abwasser 3 | Nitro-o-dichlorbenzol | kein Abbau |
| Abwasser 4 | Nitronaphthalin | 37% |

Die Abbauversuche wurden in batch-Ansätzen durchgeführt. Die Abbauwerte ergaben sich nach zweitägiger Inkubation.

Die weitere Züchtung der Kultur erfolgte in batch-Ansätzen unter Zusatz von 10% kationischem Ionenaustauscher. Nach jeweils zweitägiger Inkubation wurde das Abwasser unter Zurücklassen des Ionenaustauschers ausgetauscht. Die Abbauversuche wurden über mehrere Monate fortgesetzt. Der Abbau der Abwasserinhaltsstoffe lag auch nach vollständiger fluoretischer Beladung des Ionenaustauschers mit Abwasserinhaltsstoffen bei den geprüften Abwässern über 60%, bei den Abwässern 1, 3 und 4 über 70%.

## Patentansprüche

1. Mehrfach substituierte Kohlenwasserstoffe mit nichtidentischen Substituenten abbauende Mikroorganismen-Kulturen, bestehend aus an positive Ladungen tragenden Trägermaterialien anhaftenden Mikroorganismen, wobei die Mikroorganismen mit diesen mehrfach substituierten aromatischen Kohlenwasserstoffen als einziger Kohlenwasserstoffquelle vermehrt werden können.

2. Verfahren zur Herstellung von mindestens N-fach substituierte aromatische Kohlenwasserstoffe abbauenden Mikroorganismen-Kulturen nach Anspruch 1, dadurch gekennzeichnet, dass

a) zunächst unterschiedliche, höchstens (N − 1)-fach substituierte Kohlenwasserstoffe abbauende Mikroorganismen mit jeweils diesen (N − 1)-fach substituierten Kohlenwasserstoffen als Kohlenwasserstoffquelle getrennt vermehrt werden,

b) wobei die Substituenten der höchstens (N − 1)-fach substituierten Kohlenwasserstoffe gemeinsam mindestens alle Substituenten der N-fach substituierten Kohlenwasserstoffe mindestens einmal aufweisen,

c) die verschiedenen, an mindestens (N − 1)-fach substituierte Kohlenwasserstoffe adaptierten Kulturen vereinigt werden und durch allmählichen Austausch des Substrates durch den N-fach substituierten Kohlenwasserstoff adaptiert werden,

d) die N-fach substituierte Kohlenwasserstoffe abbauenden Mikroorganismen auf positive Ladungen tragende Trägermaterialien aufgebracht werden und

e) unter Zugabe des N-fach substituierten Kohlenwasserstoffs als Kohlenwasserstoff-Quelle weiter vermehrt werden.

3. Verfahren nach Anspruch 2, dadurch gekennzeichnet, dass N-Substituenten der mindestens N-fach substituierten Kohlenwasserstoffe anorganische Säurereste, Aminreste und/oder aliphatische Reste mit 1 bis 6 C-Atomen sind.

4. Verfahren nach einem der Ansprüche 2 oder 3, dadurch gekennzeichnet, dass die N-Substituenten nicht identisch sind.

5. Verfahren nach einem der Ansprüche 2 bis 4, dadurch gekennzeichnet, dass N = 3 oder 4 ist.

6. Verfahren nach einem der Ansprüche 2 bis 5, dadurch gekennzeichnet, dass als mindestens N-fach substituierte Kohlenwasserstoffe substituierte Naphthalinsulfonsäuren eingesetzt werden.

7. Verfahren nach einem der Ansprüche 2 bis 6, dadurch gekennzeichnet, dass die mindestens N-fach, bzw. höchstens (N − 1)-fach substituierten Kohlenwasserstoffe zusätzlich OH-, COOH- oder Aryl-Reste als Substituenten aufweisen.

8. Verfahren nach einem der Ansprüche 2 bis 7, dadurch gekennzeichnet, dass die höchstens (N − 1) der höchstens (N − 1)-fach substituierten Kohlenwasserstoffe identisch sind mit (N − 1) der mindestens N-fach substituierten Kohlenwasserstoffe.

9. Verfahren nach einem der Ansprüche 2 bis 8, dadurch gekennzeichnet, dass als positive Ladungen tragende Trägermaterialien Ionenaustauscherharze eingesetzt werden.

10. Verwendung der Mikroorganismen-Kulturen nach einem der Ansprüche 1 bis 9 in biologischen Kläranlagen.

## Claims

1. Microorganism cultures degrading multiply substituted hydrocarbons carrying non-identical substituents, consisting of microorganisms adhering to carrier materials which carry positive charges, which microorganisms can be propagated with these multiply-substituted aromatic hydrocarbons as the only source of hydrocarbons.

2. Process of the preparation of microorganisms cultures degrading at least N-times substituted aromatic hydrocarbons according to claim 1, characterised in that

a) initially different microorganisms degrading hydrocarbons which are at the most (N − 1)-times substituted are propagated separately with these (N − 1)-times substituted hydrocarbons as source of hydrocarbons,

b) the substituents of the at most (N − 1)-times substituted hydrocarbons together containing at least once all the substituents of the N-times substituted hydrocarbons,

c) the different cultures which are adapted to at least (N − 1)-times substituted hydrocarbons are combined and are adapted by gradual replacement of the substrate by the N-times substituted hydrocarbon,

d) the microorganisms which degrade N-times substituted hydrocarbon are applied to carrier materials carrying positive charges and

e) continue to be propagated with addition of the N-times substituted hydrocarbon as source of hydrocarbon.

3. Process according to claim 2, characterised in that N substituents of the at least N-times substituted hydrocarbons are inorganic acid groups, amine groups and/or aliphatic groups containing 1 to 6 carbon atoms.

4. Process according to one of the claims 2 or 3, characterised in that the N substituents are not identical.

5. Process according to one of the claims 2 to 4, characterised in that N = 3 or 4.

6. Process according to one of the claims 2 to 5, characterised in that substituted naphthalene sulphonic acids are used as at least N-times substituted hydrocarbons.

7. Process according to one of the claims 2 to 6, characterised in that the at least N-times and the at the most (N − 1)-times substituted hydrocarbons in addition contain OH-, COOH- or aryl groups as substituents.

8. Process according to one of the claims 2 to 7, characterised in that the at the most (N − 1) of the at the most (N − 1)-times substituted hydrocarbons are identical with (N − 1) of the at least N-times substituted hydrocarbons.

9. Process according to one of the claims 2 to 8, characterised in that the carrier materials used which carry positive charges are ion exchange resins.

10. Use of the microorganism cultures according to claims 1 to 9 in biological clarification plants.

**Revendications**

1. Cultures de micro-organismes dégradant des hydrocarbures polysubstitués comportant des substituants non identiques, ces cultures consistant en des micro-organismes adhérant à des matières de support portant des charges positives, les microorganismes pouvant être soumis à croissance de prolifération en utilisant ces hydrocarbures aromatiques polysubstitués comme seule source d'hydrocarbures.

2. Procédé pour préparer des cultures de micro-organismes dégradant des hydrocarbures aromatiques au moins n fois substitués, selon la revendication 1, procédé caractérisé en ce que:

a) on soumet tout d'abord à croissance de prolifération séparée des micro-organismes différents, dégradant des hydrocarbures substitués au maximum (n − 1) fois, en utilisant à chaque fois ces hydrocarbures substitués (n − 1) fois comme source d'hydrocarbures,

b) les substituants des hydrocarbures substitués au maximum (n − 1) fois présentant ensemble au moins une fois tous les substituants des hydrocarbures substitués n fois,

c) on groupe les cultures différentes, adaptées à des hydrocarbures au moins (n − 1) substitués et, par remplacement progressif du substrat par l'hydrocarbure substitué n fois, on les adapte à cet hydrocarbure,

d) on applique sur des matières de support, portant des charges positives, les micro-organismes dégradant des hydrocarbures n fois substitués et,

e) en ajoutant l'hydrocarbure n fois substitué comme source d'hydrocarbure(s), on provoque la poursuite de la croissance de prolifération de ces cultures.

3. Procédé selon la revendication 2, caractérisé en ce que n substituants des hydrocarbures au moins n fois substitués sont des restes d'acides minéraux, des restes d'amines et/ou des restes aliphatiques comportant 1 à 6 atomes de carbone.

4. Procédé selon l'une des revendications 2 ou 3, caractérisé en ce que les n substituants ne sont pas identiques entre eux.

5. Procédé selon l'une des revendications 2 à 4, caractérisé en ce que n vaut 3 ou 4.

6. Procédé selon l'une des revendications 2 à 5, caractérisé en ce qu'on utilise, comme hydrocarbures substitués au moins n fois, des acides naphthalène-sulfoniques substitués.

7. Procédé selon l'une des revendications 2 à 6, caractérisé en ce que les hydrocarbures substitués au moins n fois, ou substitués au maximum (n − 1) fois, présentent en outre des restes −OH, −COOH ou aryle comme substituants.

8. Procédé selon l'une des revendications 2 à 7, caractérisé en ce qu'au maximum (n − 1) des hydrocarbures substitués au maximum (n − 1) fois sont identiques à (n − 1) hydrocarbures substitués au moins n fois.

9. Procédé selon l'une des revendications 2 à 8, caractérisé en ce qu'on utilise, comme matières de support portant des charges positives, des résines d'échange d'ions.

10. Utilisation des cultures de micro-organismes selon l'une des revendications 1 à 9 dans des installations de clarification biologique.

FIG. 1

Chromatogramm II
Cleve-Säure

Chromatogramm III

Chromatogramm IV

Chromatogramm V

Chromatogramm I

FIG. 2